# EUROPEAN PATENT APPLICATION

(11) **EP 4 782 004 A1**
(43) Date of publication of application: **29.07.2026**
(21) Application number: 24867622.3
(22) Date of filing: 20.09.2024
(51) Int. Cl.: A61K 38/48, A61P 1/14

(54) **GLUTEN-DEGRADING PROTEIN AND USES THEREOF**

(30) Priority: 22.09.2023 ES 202330796
(71) Applicant: Consejo Superior de Investigaciones Científicas (CSIC), 28006 Madrid (ES); Universitat de Barcelona, 08007 Barcelona (ES)
(72) Inventor: GOMIS-RÜTH, Francesc Xavier, 08028 Barcelona Barcelona (ES); DEL AMO MAESTRO, Laura, 08028 Barcelona Barcelona (ES); DOS MENDES REI, Soraia Ines, 08028 Barcelona Barcelona (ES); ECKHARD, Ulrich, 08028 Barcelona Barcelona (ES); RODRÍGUEZ BANQUERI, Arturo, 08028 Barcelona Barcelona (ES); GUEVARA PUIG, Tibisay, 08028 Barcelona Barcelona (ES); PÉREZ CANO, Francisco, 08028 Barcelona (ES); RODRÍGUEZ LAGUNAS, María José, 08028 Barcelona (ES); FRANCH MASFERRER, Àngels, 08028 Barcelona (ES); GIRBAL GONZALEZ, Marina, 08028 Barcelona (ES)
(74) Representative: Isern Patentes y Marcas S.L.
(86) International application number: PCT/ES2024/070570
(87) International publication number: WO 2025/062056

(57) **Abstract**

The present invention relates to a protein comprising the amino acid sequence SEQ ID NO: 1, compositions comprising same, as well as uses thereof, in the treatment of diseases caused by the presence of peptide antigens present in gluten, in the treatment of gluten-containing foods or in the preparation of gluten-free food products.

## Description

The present invention is within the field of biomedicine and food biotechnology. Specifically, the present invention relates to a protein comprising the amino acid sequence SEQ ID NO: 1, as well as thereof, as the treatment of diseases caused by the presence of peptide antigens present in gluten or in the pre-treatment of gluten-containing foods

### STATE OF THE ART

Gluten proteins (gliadins and prolamins and glutenin analogues) are the main environmental factors that trigger celiac disease and other associated disorders. These proteins contain peptide sequences rich in proline and glutamine, which makes them more resistant to digestive enzymes than other dietary proteins, which may persist in the intestinal lumen. In susceptible individuals, these peptides are responsible for an abnormal reaction involving both innate and adaptive immunity and which, overall, causes chronic inflammation of the intestinal mucosa, an increase in intraepithelial lymphocytes, crypt hyperplasia and progressive deterioration of the intestinal villi, and even their complete disappearance.

Disorders associated with gluten intake, such as celiac disease, are becoming a major public health problem worldwide. In fact, although celiac disease was previously believed to occur only, or predominantly, in northern and western Europe, it is now recognised to be present throughout the world. A systematic review of the global prevalence of celiac disease found a seroprevalence rate of 1.4%, with prevalence varying by continent between 1.3% (South America, 11 studies) and 1.8% (Asia, 20 studies). Singh P. et al. Global Prevalence of Celiac Disease: Systematic Review and Meta-analysis. Clin Gastroenterol Hepatol. 2018 Jun;16(6):823-836. Other associated disorders include non-celiac gluten sensitivity (NCGS) and irritable bowel syndrome (IBS), which together affect more than 13% of the world's population.

Some strategies aimed at improving the health of individuals with disorders associated with gluten intake include the use of microorganisms such as probiotics. ES2343499B1 describes the use of a strain of the species *Bifidobacterium longum* to improve the health status of patients with related diseases. Another known approach or strategy is the use of microorganisms capable of hydrolysing immunotoxic peptides in bread doughs (DiCagno et al. 2004). Sourdough bread made from wheat and nontoxic flours and selected lactobacilli is tolerated in celiac sprue patients. Appl Environ Microbiol 70:1088-96).

Moreover, the use of recombinant proteins for the treatment and/or prevention of disorders associated with gluten intake has also been described in the state of the art. Patent documents such as ES2883347T3 or WO2015192211A1, belonging to the same patent family, describe compositions and methods for the attenuation or prevention of intestinal inflammation caused by the presence of peptide food antigens based on the use of an enzyme called neprosin, capable of carrying out the digestion of gliadins.

Piper et al. 2004 (Effect of prolyl endopeptidase on digestive-resistant gliadin peptides in vivo. J Pharmacol Exp Ther.311:213-9) or Tack et al. 2013 (Consumption of gluten with gluten-degrading enzyme by celiac patients: a pilot-study. World J Gastroenterol. 19(35):5837-47; Gass *et al.* 2007) disclose the hydrolysis of immunotoxic peptides from gluten during the digestive process, preventing them from reaching the small intestine by means of oral administration of other purified enzymes of plant or bacterial origin capable of degrading gluten peptides (Piper et al. 2004. Effect of prolyl endopeptidase on digestive-resistant gliadin peptides in vivo. J Pharmacol Exp Ther.311:213-9; Tack et al. 2013. Consumption of gluten with gluten-degrading enzyme by celiac patients: a pilot-study. World J Gastroenterol. 19(35):5837-47; Gass *et al.* 2007).

However, despite the severity of the symptoms and the high prevalence of celiac disease, the development of effective therapies is limited, and completely avoiding gluten consumption is a complicated task because traces of gluten are found in more than 80% of all foods consumed.

Therefore, there is a need for alternative and preferably improved treatments compared to those described in the state of the art, as well as alternative strategies aimed at processing and preparing gluten-free or low-gluten food products.

### DESCRIPTION OF THE INVENTION

As explained in the background section, an enzyme known as neprosin, derived from *Nepenthes ventrata,* is known in the state of the art and is capable of digesting gliadins (which, as will be described later in this document, are a group of proteins present in gluten), the amino acid sequence of which is as follows:

MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGNASLQGANATINIWEPNLKNPNGDFSLTQIWI
SAGSGSSLNTIEAGWQVYPGRTGDSQPRFFIYWTADGYTSTGCYDLTCPGFVQTNN
YYAIGMALQPSVYGGQQYELNESIQRDPATGNWWLYLWGTVVGYWPASIYNSITNG
ADTVEWGGEIYDSSGTGGFHTTTQMGSGHFPTEGYGKASYVRDLQCVDTYGNVISP
TANSFQGIAPAPNCYNYQFQQGSSELYLFYGGPGCQ (SEQ ID NO: 2)

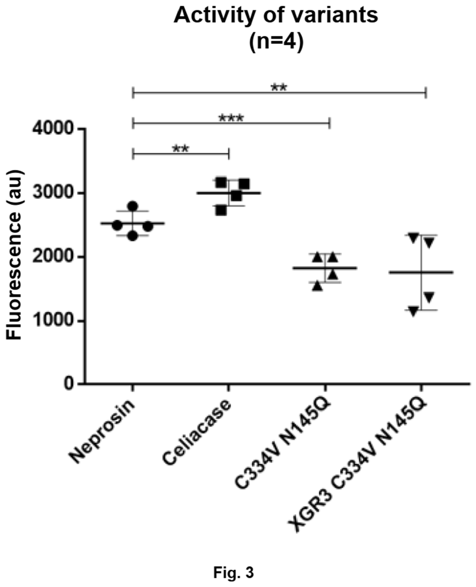

The inventors of the present invention have designed and obtained a protein in recombinant form, referred to in the present invention as celiacase or protein of the invention, the sequence of which, SEQ ID NO: 1, exhibits the C334V mutation with respect to the sequence of the Neprosin protein. Said mutation was designed by the inventors upon determining the crystallographic structure of Neprosin and finding that the cysteine at position 334 could be responsible for the recurring yield losses observed in the production, purification and activation of the enzyme. In a series of assays, the inventors have demonstrated that celiacase is more effective than neprosin in performing a cleavage reaction of the QPQL sequence (SEQ ID NO: 3), which is part of peptides present in gluten such as 33-mer (LQLQPFPQPQLPYPQPQLPYPQPQLPYPQPQPF; SEQ ID NO: 4), as shown in Fig. 3.

The genetic modification made in the enzyme thus increased the enzyme production yield and also the activity and capacity for gluten degradation, specifically gliadins and gliadin-derived peptides which cannot be degraded in normal digestion, with the main components being associated with celiac disease, having different applications of interest as will be described below in the present specification.

Furthermore, they have also experimentally demonstrated that among all mutants generated from neprosin, celiacase is the protein that showed the best activity, stability and performance (Fig. 3).

### Protein of the invention

One aspect of the invention relates to a protein, comprising, preferably consisting of, the amino acid sequence SEQ ID NO: 1, hereinafter the "protein of the invention", also referred to as celiacase.

SEQ ID NO: 1
MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGNASLQGANATINIWEPNLKNPNGDFSLTQIWI
SAGSGSSLNTIEAGWQVYPGRTGDSQPRFFIYWTADGYTSTGCYDLTCPGFVQTNN
YYAIGMALQPSVYGGQQYELNESIQRDPATGNWWLYLWGTVVGYWPASIYNSITNG
ADTVEWGGEIYDSSGTGGFHTTTQMGSGHFPTEGYGKASYVRDLQVVDTYGNVISP
TANSFQGIAPAPNCYNYQFQQGSSELYLFYGGPGCQ

In the context of the present invention, protein is understood to be a molecule formed by the attachment of amino acids by means of peptide bonds. In a preferred embodiment, the protein of the invention has a length of 380 to 500 amino acids (including the ends).

The protein of the invention can be obtained by techniques widely known in the state of the art. Examples of techniques for obtaining proteins include, but are not limited to, chemical or biological synthesis, genetic recombination, or expression of polynucleotides encoding the protein of the invention.

Furthermore, once obtained, the protein of the invention may be subjected to a lyophilisation process. Lyophilisation is a technique or process known in the state of the art, carried out under vacuum and at low temperature to avoid protein denaturation. Therefore, in a preferred embodiment, the protein of the invention is lyophilised.

The protein of the invention may further comprise a his-tag sequence at the C-terminus to facilitate its purification (to which three amino acid spacers, such as AIA, may be added, giving rise to the sequence AIAHHHHHH (SEQ ID NO: 5)). Therefore, in a preferred embodiment, alone or in combination with other preferred embodiments, the protein of the invention further comprises the sequence SEQ ID NO: 5.

Furthermore, the protein of the invention can be bound/linked to a signal peptide that directs the protein to the secretory pathway in a cell. Therefore, in a preferred embodiment, alone or in combination with other preferred embodiments, the protein of the invention further comprises a signal peptide or signal sequence. Techniques for adding signal peptides to the protein of interest are known in the state of the art.

Signal peptide sequences effective for bacterial host cells are the signal peptide obtained from maltogenic amylase from *Bacillus stearothermophilus, B. licheniformis* subtilisin, *B*. *licheniformis* beta-lactamase, *Bacillus stearothermophilus* alpha-amylase, *B*. *stearothermophilus* neutral proteases (nprT, nprS, nprM), *Bacillus subtilis* prsA, etc. Signal peptide sequences effective for filamentous fungal host cells are the signal peptide obtained from neutral amylase from *Aspergillus niger, A. niger* glucoamylase A, *Aspergillus shirousami* glucoamylase A, *Aspergillus kawachii* glucoamylase A, *Aspergillus oryzae* TAKA amylase, *Humicola insolens* cellulase, *H. insolens* endoglucanase V, *Humicola lanuginosa* lipase and *Rhizomucor miehei* aspartic proteinase.

Techniques for producing fusion polypeptides, for example, for adding signal peptides to the protein of interest, are known in the art and include linking the coding sequences encoding the polypeptides so that they are in-frame and expression of the fusion polypeptide is under the control of the same promoter(s) and terminator. Fusion polypeptides can also be constructed using intein technology in which fusion polypeptides are created post-translationally.

### Polynucleotide, gene constructs and cell of the invention

As indicated above, the protein of the invention can be obtained by techniques widely known in the state of the art, such as the expression in a cell of the polynucleotide encoding the protein of the invention, and the subsequent isolation thereof. Therefore, in another aspect, the invention relates to a polynucleotide, hereinafter the "polynucleotide of the invention", encoding the protein of the invention.

The term "polynucleotide", as used in the present invention, refers to a polymeric form of nucleotides of any length, formed by ribonucleotides and/or deoxyribonucleotides. The term includes both single-stranded and double-stranded polynucleotides, as well as modified polynucleotides (methylated, protected and similar). The polynucleotide of the invention may be a DNA, an RNA, or a cDNA.

The polynucleotide of the invention can be obtained by a skilled person by means of using techniques widely known in the state of the art. The sequence of the polynucleotide of the invention may be comprised in a gene construct.

Therefore, in another aspect, the invention relates to a gene construct, hereinafter the "gene construct of the invention", which comprises the polynucleotide of the invention.

Preferably, the construct comprises the polynucleotide of the invention operably linked to regulatory sequences for the expression of the polynucleotide of the invention. In theory, any promoter can be used in the gene constructs of the present invention, provided that said promoter is compatible with the cells in which the polynucleotide is to be expressed, the cells preferably being human cells, most preferably Expi293F cells.

A promoter, or promoter region, is a nucleotide sequence that controls the transcription of a given gene (nucleotide sequences). The present invention relates to a nucleotide sequence that controls the transcription of the polynucleotide of the invention. Promoter sequences can be unidirectional or bidirectional. A unidirectional promoter is one that controls the transcription of one or more genes that are located in tandem with the first. "In tandem" refers to the 3' end of the first gene that is followed, either consecutively or separated by a particular nucleotide sequence, by the 5' end of the second gene. A bidirectional promoter refers to the promoter region that controls transcription in two opposite directions. In other words, a bidirectional promoter directs the transcription of two divergently located genes, i.e., in the opposite direction, where the 5' end of both nucleotide sequences are closer to each other than the 3' end. In the present invention, the terms "promoter" and "promoter region" are used interchangeably.

Other appropriate sequences that may be comprised by the gene construct are sequences that control and regulate said transcription and, if applicable, the translation of the product of interest, such as, for example, transcription initiation and termination signals, polyadenylation signal, origin of replication, or ribosome binding sequences (RBS), coding sequences of transcriptional regulators, among others.

Additionally, the gene construct of the invention may contain markers or tags that allow the protein of the invention to be isolated once it is synthesised in the cell.

Moreover, the polynucleotide of the invention or gene construct comprising same may further comprise a coding sequence for a signal peptide. Therefore, in a preferred embodiment, the polynucleotide or gene construct of the invention further comprises a coding sequence for a signal peptide, preferably, wherein said sequence is bound to the polynucleotide of the invention. Signal peptides that can be bound/linked to the protein of the invention have been described above in the present document.

Moreover, the polynucleotide or the gene construct of the invention may be part of a vector. Therefore, in another aspect, the invention relates to a vector, hereinafter "vector of the invention", which comprises the polynucleotide or the gene construct of the invention.

Examples of suitable expression vectors can be selected according to the conditions and needs of each specific case. The choice of the vector will depend on the host cell and on the type of intended use. Said vector can be obtained by conventional methods known to a man of the art, such as, but not limited to, chemical transformation, electroporation, microinjection, among others, if the host cell is a eukaryote. Examples of suitable vectors in the context of the present invention include, but are not limited to, the pET-28a(+) vector or the pCMV vector.

The vector of the invention can be used, for example, to transform, transfect, or infect cells susceptible to being transformed, transfected, or infected by said vector. In the context of the present invention, preferably, the cells are human and are transfected with the vector of the invention.

Therefore, another aspect of the invention relates to a cell, preferably a human cell, even more preferably Expi293F human cells, hereinafter the "cell of the invention", comprising the protein, the polynucleotide, the gene construct, or the vector of the invention, for which said cell has been able to be transformed, transfected, transduced or infected with a construct or a vector provided by this invention. Transformed, transfected, transduced or infected cells can be obtained by conventional methods known to those skilled in the art. In a preferred embodiment, said host cell is transfected with an appropriate vector.

The term "expression" includes any step involved in the production of the protein of the invention including, but not limited to, transcription, post-transcriptional modification, translation, post-translational modification and secretion.

Moreover, as understood by one skilled in the art, the protein of the invention can be purified by means of a variety of methods known in the art including, but not limited to, chromatography (for example, ion exchange, affinity, hydrophobic, chromatofocusing and size exclusion), electrophoretic methods (for example, preparative isoelectric focusing), differential solubility (for example, precipitation with ammonium sulphate), SDS-PAGE or extraction, in order to obtain substantially pure celiacase, which can be included, for example, in a composition.

### Composition of the invention and derived formulations

Likewise, the protein, the polynucleotide, the gene construct, the vector, or the cell of the invention can be part of a composition.

Therefore, another aspect of the invention relates to a composition, hereinafter, the "composition of the invention", comprising the protein, the polynucleotide, the gene construct, the vector, or the cell of the invention.

In a preferred embodiment, the composition of the invention is a pharmaceutical composition.

In the present invention, "pharmaceutical composition" is understood to be any pharmaceutical preparation or form, the composition formula of which expressed in units of the international system consists of a substance or mixture of substances, with constant weight, volume and percentages, produced in legally established pharmaceutical laboratories, packaged, or labelled to be distributed and marketed as effective for diagnosis, treatment, mitigation, and prophylaxis of a disease, physical anomaly, or symptom, or the restoration, correction, or modification of the balance of organic functions in humans and animals. The pharmaceutical composition can be prepared by any of the methods described in the state of the art.

In another preferred embodiment, the composition of the invention further comprises a carrier, which in the case of a pharmaceutical composition, will be a pharmaceutically acceptable carrier.

The term "carrier" refers to a diluent or excipient with which the active ingredient is administered. Such carriers may be sterile liquids, such as water and oils, including those of petroleum, animal, plant, or synthetic origin, such as peanut oil, soya oil, mineral oil, sesame oil, and the like. Water or aqueous saline solutions and aqueous dextrose and glycerol solutions are preferably used as carriers, particularly for injectable solutions. Preferably, in the case of pharmaceutical carriers, they are approved by a state or federal government regulatory agency or are listed in the United States Pharmacopeia or other generally recognised pharmacopeia for use in animals, and more particularly in humans. The carriers and auxiliary substances necessary to manufacture the desired pharmaceutical form of administration of the pharmaceutical composition of the invention will depend, among other factors, on the chosen pharmaceutical form of administration. Said pharmaceutical forms of administration of the pharmaceutical composition will be manufactured according to conventional methods known to the person skilled in the art.

In a preferred embodiment, the composition of the invention comprises the protein, the polynucleotide, the gene construct, the vector or the cell of the invention in a therapeutically effective amount.

In the sense used in this description, the expression "therapeutically effective amount" refers to the amount of the active agent or ingredient, or of the pharmaceutical composition of the invention, that produces the desired effect and, in general, will be determined, among other causes, by the characteristics of said compound or said pharmaceutical composition and the therapeutic effect to be achieved. The dose for obtaining a therapeutically effective amount depends on a variety of factors such as, for example, age, weight, sex, or tolerance, of the mammal. The "adjuvants" and "pharmaceutically acceptable carriers" that can be used in said compositions are the carriers known in the state of the art.

In a preferred embodiment, the concentration of the protein of the invention in the composition of the invention is from 0.000001 to 500 mg/ml (including end values). In a more preferred embodiment, the concentration of the protein of the invention in the composition of the invention is from 0.00001 mg/ml to 200 mg/ml (including end values) or from 0.0001 mg/ml to 100 mg/ml (including end values).

Additionally, the composition of the invention may comprise an adjuvant. "Adjuvant" is understood to be any substance that enhances the effectiveness of the pharmaceutical composition of the invention. Examples of adjuvants include, but are not limited to, adjuvants formed by aluminium salts (alum), such as aluminium hydroxide, aluminium phosphate or aluminium sulphate, oil-in-water or water-in-oil emulsion formulations such as Freund's Complete Adjuvant (FCA) as well as Freund's Incomplete Adjuvant (FIA), mineral gels; block copolymers, Avridine^{™}, SEAM62, adjuvants formed by components of the bacterial cell wall such as adjuvants including liposaccharides (e.g., lipid A or monophosphoryl lipid A (MLA), trehalose dimycolate (TDM), and components of the cell wall skeleton (CWS)), heat shock proteins or derivatives thereof, adjuvants derived from ADP-ribosylating bacterial toxins, including diphtheria toxin (DT), pertussis toxin (PT), cholera toxin (CT), *E. coli* heat-labile toxins (LT1 and LT2), *Pseudomonas* endotoxin A and exotoxin, *B. cereus* exoenzyme B, *B. sphaericus* toxin, *C. botulinum* toxins C2 and C3, *C. limosum* exoenzyme as well as the toxins of *C*. *perfringens, C. spiriforma* and *C*. *difficile, S*. *aureus,* EDIM, and mutants of mutant toxins such as CRM-197, non-toxic mutant of diphtheria toxin; saponins such as ISCOMs (immunostimulating complexes), chemokines, chemokines and cytokines such as interleukins (IL-1, IL-2, IL-4, IL-5, IL-6, IL-7, IL-8, IL-12, etc.), interferons (such as interferon gamma), macrophage colony-stimulating factor (M-CSF), tumour necrosis factor (TNF), defensins 102, RANTES, MIPI-alpha, and MEP-2, muramyl peptides; adjuvants derived from the family of CpG molecules, CpG dinucleotides and synthetic oligonucleotides comprising CpG motifs, *C*. *limosum* exoenzyme and synthetic adjuvants such as PCPP, cholera toxin, Salmonella toxin, alum, and the like, aluminium hydroxide, N-acetyl-muramyl-L-threonyl-D-isoglutamine (thr-MDP), N-acetyl-nor-muramyl-L-alanyl-D-isoglutamine, MTP-PE and RIBI, containing three components extracted from bacteria, monophosphoryl lipid A, trehalose dimycolate and cell wall skeleton (MPL+TDM+CWS) in an emulsion of 12% squalene/Tween 80. Other examples of adjuvants include DDA (dimethyldioctadecylammonium bromide), QuilA or Freund's complete and incomplete adjuvants.

Furthermore, the composition of the invention may comprise other additional active or inactive components. Additional components include salts to modulate ionic strength, preservatives including, but not limited to, antimicrobial agents, antioxidants, chelating agents, or the like, or nutrients, including glucose, dextrose, vitamins, and minerals. Other components, such as carriers or inert excipients, include, without being limited to, binders; excipients; dispersing agents, such as alginic acid; lubricants, such as magnesium stearate, glidants such as colloidal silicon dioxide; sweetening agents; or flavouring agents, such as mint or methyl salicylate.

In a preferred embodiment, alone or in combination with other preferred embodiments, the composition of the invention further comprises binders.

Binders are used in solid dosage forms, e.g., to keep the ingredients in one tablet together, to ensure that tablets and granules can be formed with the required mechanical strength, and to provide bulk to low active dose tablets. Binders in solid dosage forms such as tablets include: lactose, sucrose, corn (maize) starch, modified starches, microcrystalline cellulose, modified cellulose (for example, hydroxypropyl methylcellulose (HPMC) and hydroxyethylcellulose), other water-soluble cellulose ethers, polyvinylpyrrolidone (PVP) also known as povidone, polyethylene glycol, sorbitol, maltitol, xylitol and dibasic calcium phosphate; or mixtures of these substances.

The term "pharmaceutically acceptable" refers to the fact that the carrier or excipient must allow the activity of the compounds in the pharmaceutical composition, in particular of the protein of the invention, in other words, it is compatible with said components, so that it does not cause harm to the organisms to which it is administered.

Said compositions and/or their formulations may be administered to an animal, including a mammal and, therefore, a human, orally. Therefore, in a preferred embodiment, alone or in combination with other preferred embodiments, the composition of the invention is formulated for oral administration.

Moreover, the composition of the invention can be formulated in solid, semi-solid or liquid preparations. In a preferred embodiment, the composition of the invention is formulated in solid form or liquid form.

**In** a more preferred embodiment, the solid formulation is selected from the list consisting of tablets, pills, chewable tablets, capsules (including hard-shell type capsules), sachets, powders, gels, granules, coated particles, dispersible strips and films.

In another more preferred embodiment, the liquid formulation is selected from the list consisting of oral solutions, suspensions, emulsions, drops and syrup.

Furthermore, the composition of the invention may be, in certain embodiments, buffered.

As mentioned above, the protein of the invention needs an acidic pH, similar to that of the stomach, to switch to its active form.

Therefore, in some embodiments of the present invention, the composition comprises a suitable buffer, such that the pH of the composition is less than 6, preferably such that the pH of the composition is 3 to 5.

Examples of suitable tampons include, but are not limited to, 100 mM glycine pH 2.5, 100 mM glycine pH 3, 20 mM HCl pH 2.5, 20 mM HCl pH 3. Therefore, in a more preferred embodiment, the buffer is selected from the list consisting of 100 mM glycine buffer pH 2.5, 100 mM glycine buffer pH3, 20 mM HCl buffer pH 2.5 and 20 mM HCl buffer pH 3.

Other aspects of the invention relate to a pharmaceutical product, a veterinary product, a medical food, a food product, a food ingredient or additive, a food supplement, a probiotic food composition, a dietary supplement, a symbiotic composition, or a biotherapeutic composition, comprising an effective amount of the composition of the invention, preferably, further comprising appropriate quantities of acceptable excipients.

In the context of the present invention, the products mentioned above are tolerated by a celiac subject, comprising an effective amount of the composition of the invention. The expression "tolerated by a celiac subject", as used herein, refers to the fact that said products are suitable for celiac subjects, being gluten-free, or containing reduced or trace levels of gluten, that therefore allow them to be tolerated by subjects suffering from this disorder.

"Probiotic food composition" is understood to be any combination of ingredients, which composition is a constituent of a gluten-free solid or liquid food product, which, in addition to its nutritional characteristics, includes specific characteristics that help improve health and reduce the risk of disease.

"Food supplement" is understood to be an additional nutritional supplement intended to supplement the diet.

"Biotherapeutic composition" is understood to be a product with a beneficial effect on health or a therapeutic effect.

"Symbiotic composition" is understood to be a functional food containing a mixture of prebiotic and probiotic food products.

The term "pharmaceutical product" is understood in its broad meaning in this description, without being limited to a medicinal product, including any composition comprising an active ingredient, in this case, the protein and/or derived aspects such as the cell of the invention, together with pharmaceutically acceptable excipients/carriers.

The term "food product" is used in the present specification in its broadest meaning, including any type of product, in any form of presentation, which can be ingested by an animal, but excluding pharmaceutical and veterinary products. Examples of food products include, but are not limited to, dairy products (a yoghurt, a cheese, a fermented milk, a milk powder, a fermented milk-based product, an ice cream, a fermented cereal-based product, a milk-based powder), bread, bars, spreadable products, biscuits and cereals, a beverage, different types of oil, or a dressing. Examples of other food products include meat products (e.g., sausages or spreadable meat products), chocolate spreads, fillings and glazes, chocolate, pastries, baked goods (buns, puff pastries), sauces and soups, fruit juices and coffee whiteners.

In a preferred embodiment, the food product is selected from the list consisting of yoghurt, cheese, fermented milk, powdered milk, fermented milk-based product, ice cream, a fermented cereal-based product, milk-based powder, bread, bars, spreadable products, biscuits, cereals, beverages, oil, sausages, spreadable meat products, chocolate spreads, fillings and glazes, chocolate, baked goods, including buns or puff pastries, sauces and soups. More preferably, the food product is bread or chocolate.

"Food ingredient" is understood to be any substance used in the manufacture or preparation of a food product and still present in the finished product, albeit in a modified form. In a particular embodiment, the food ingredient is flour.

"Additive" is understood to be substances that are intentionally added to a food during its manufacturing or preparation process in order to change, intensify or maintain its colour, texture, flavour, shelf life, etc. In a preferred embodiment, the additive is a preservative.

The terms "medical food" or "food for special medical purposes" refer to a specially formulated food intended for the dietary management of a disease that has distinctive nutritional needs that cannot be met by a normal diet alone. Medical foods are distinct from the broader category of food supplements and traditional foods that exhibit a health claim.

The term "dietary supplement" can be defined as a preparation or product intended to supplement the diet, prepared from compounds commonly used in food products, that provides beneficial nutrients or ingredients that are not usually ingested in the normal diet or that may not be consumed in sufficient quantities.

The term "effective amount" refers to any amount of the composition of the invention which, when administered to a subject, is sufficient to produce the desired effect.

### Therapeutic uses of the invention

As explained herein above, the protein of the invention, as well as the cell or composition of the invention, preferably the pharmaceutical composition of the invention, exhibit different activities with potential therapeutic use in subjects.

Therefore, another aspect of the invention relates to the protein, the cell, or the composition of the invention for use as a medicinal product.

In another aspect, the invention relates to the use of the protein, the cell, or the composition of the invention, in the preparation of a medicinal product or pharmaceutical composition.

The term "medicinal product", as it is used herein, refers to any composition/substance used for the prevention, diagnosis, relief, treatment, or cure of diseases in a subject or which can be administered to the subject in order to restore, correct, or modify their physiological functions by exerting a pharmacological, immunological, or metabolic action.

In another aspect, the invention relates to the protein, the cell, or the composition of the invention for use in the prevention, attenuation and/or treatment of a disease in a subject due to the presence of peptide antigens, wherein the peptide antigen is present in gluten.

In another aspect, the invention relates to the use of the protein, the cell, or the composition of the invention in the preparation of a medicinal product or pharmaceutical composition for the prevention, attenuation and/or treatment of a disease in a subject due to the presence of peptide antigens, wherein the peptide antigen is present in gluten.

In another aspect, the invention relates to a method for the prevention, attenuation and/or treatment of a disease in a subject due to the presence of peptide antigens, wherein the peptide antigen is present in gluten, which comprises the administration to said subject of the protein, the cell, or the composition of the invention.

In the present invention, "treatment" is understood to be the set of means used to treat or cure a disease.

The term "prevention" can be defined the set of means or measures aimed to prevent the onset of a disease, illness, or disorder, or to delay its progress.

The term "attenuation" can be defined as alleviating the disease or disorder, in other words, causing the regression of abnormal biological reactions and/or symptoms.

In the present invention, "subject" is understood to be any animal, preferably a mammal, more preferably a primate, in particular, a human, of any race, sex, or age. Therefore, in a preferred embodiment, the subject is a mammal, preferably a human being. In another preferred embodiment, alone or in combination with other preferred embodiments, the subject suffers from celiac disease and/or disorders associated with gluten intake.

As used in this specification, the term "gluten" generally refers to proteins present in wheat or related grain species, including barley and rye, that have a potentially detrimental effect for certain individuals. Gluten proteins include gliadins, such as α-gliadins, β-gliadins, γ-gliadins and ω-gliadins, which are monomeric proteins, and glutenins, which are highly heterogeneous mixtures of aggregates of high molecular weight and low molecular weight subunits bound by means of disulphide bonds. Many wheat gluten proteins have been characterised. See, for example, Woychik et al., Amino Acid Composition of Proteins in Wheat Gluten, J. Agric. Food Chem., 9 (4), 307-310 (1961). The term gluten, as used in this specification, also includes oligopeptides that can be obtained from normal human digestion of gluten proteins from gluten-containing foods which cause the abnormal immune response. Some of these oligopeptides are resistant to normal digestive enzymes. It is believed that gluten, including the proteins and oligopeptides mentioned above, acts as an antigen for T-lymphocytes in susceptible patients (for example, in patients with celiac disease or gluten intolerance). The term gluten also refers to denatured gluten, as would be found in baked goods.

The term "peptide antigen" in the present invention relates to a molecule consisting of an amino acid chain that has the ability to trigger an immune response in an organism. Specifically, antigens present a region, portion or fragment known as an epitope or antigenic determinant, which is recognised by the immune system, triggering an immune response. Antigenic peptides are presented on the surface of antigen-presenting cells, such as dendritic cells, together with major histocompatibility complex (MHC) molecules. This presentation allows lymphocytes to recognise and bind specifically to the presented peptide antigen, leading to activation of the immune response.

In a preferred embodiment, alone or in combination with other preferred embodiments, the peptide antigen is a food antigen.

In another preferred embodiment, alone or in combination with other preferred embodiments, the disease is a bowel disease.

In another preferred embodiment, alone or in combination with other preferred embodiments, the peptide antigen is rich in proline and/or glutamine residues.

In another preferred embodiment, alone or in combination with other preferred embodiments, the peptide antigen is present on a gliadin or a fragment thereof, preferably wherein the gliadin is an α-gliadin
The primary structure of gliadins exhibits a high percentage of proline and glutamine residues, which makes it highly resistant to degradation by gastrointestinal enzymes. Because of that, peptides resistant to the action of gastrointestinal enzymes accumulate in the intestinal lumen (Shan et al. 2005. Identification and analysis of multivalent proteolytically resistant peptides from gluten: implications for celiac sprue. J Proteome Res. 4, 1732-1741). Among them, there is a 33-amino acid (33-mer) peptide (LQLQPFPQPQLPYPQPLPYPQPQLPYPQPQPF; SEQ ID NO: 4) which has been described as the most immunogenic peptide in gluten, by possessing six overlapping toxic epitopes (Qiao et al. 2004. Antigen presentation to celiac lesion-derived T cells of a 33-mer gliadin peptide naturally formed by gastrointestinal digestion. J Immunol 173:1757-1762). In susceptible individuals, these peptides are responsible for an abnormal reaction that eventually gives rise to chronic inflammation of the intestinal mucosa, crypt hyperplasia and progressive deterioration of the intestinal villi, and even their complete disappearance.

In another preferred embodiment, alone or in combination with other preferred embodiments, the peptide antigen, the peptide antigen comprises the amino acid sequence SEQ ID NO: 3. More preferably, the antigen comprises the amino acid sequence SEQ ID NO: 4, which in turn comprises the amino acid sequence SEQ ID NO: 3.

In another preferred embodiment, alone or in combination with other preferred embodiments, the peptide antigen is 33-mer of α-gliadin or a fragment thereof.

Since the protein of the invention is capable of cleaving the QPQL amino acid sequence (SEQ ID NO: 3) which is part of the peptides that generate the celiac disease response in celiac patients, the protein of the invention, the cell, or the composition of the invention, preferably wherein the composition of the invention, the pharmaceutical composition of the invention, have an application in the prevention, attenuation, and/or treatment of celiac disease and/or disorders associated with gluten intake.

Therefore, in a preferred embodiment, the disease is celiac disease and/or disorders associated with gluten intake.

Celiac disease can be defined as a chronic autoimmune disorder that is induced by, or the main triggering factor of which is, the intake of gluten in genetically predisposed individuals. The typical symptomatology is associated with intestinal disturbances, due to atrophy of the intestinal villi and crypt hyperplasia, which cause a drastic reduction in the surface area of the intestinal wall (malabsorption), diarrhoea, anaemia, fatigue, weight loss, abdominal distention, etc.).

The term "celiac disease and/or disorders associated with gluten intake", includes but is not limited to any condition or disorder triggered by gluten proteins or peptides, including, but not limited to, gluten intolerance, wheat allergy, gluten sensitivity, gluten-sensitive enteropathy, idiopathic gluten sensitivity and dermatitis herpetiformis.

In many cases it may further have associated extra-intestinal symptoms (osteoporosis, depression, infertility, etc.).

Furthermore, gluten intake and celiac disease are closely associated with other disorders such as, but not limited to, type I diabetes mellitus, Down's syndrome, multiple sclerosis, dermatitis herpetiformis, myopathy, arthritis, including rheumatoid arthritis, autism, schizophrenia, lymphomas, attention deficit hyperactivity disorder (ADHD), fibromyalgia, Crohn's disease, nutrient malabsorption and irritable bowel syndrome (IBS).

In a preferred embodiment, alone or in combination with other preferred embodiments, the protein, composition, or cell of the invention is administered to the subject prior to the ingestion of a potentially antigenic food or protein containing gluten. In another preferred embodiment, the protein, composition, or cell of the invention is administered to the subject with the ingestion of a potentially antigenic food or protein containing gluten. In another preferred embodiment, the protein, composition, or cell of the invention is administered to the subject after the ingestion of a potentially antigenic food or protein containing gluten. In another preferred embodiment, the protein, composition, or cell of the invention is administered to the subject independently of the consumption of a potentially antigenic food or protein.

### Industrial uses

In addition to the aforementioned therapeutic uses, the protein of the invention, as well as the composition or cell of the invention, have other applications of interest, derived from the ability of the protein of the invention to carry out cleavage of peptide sequences comprising the QPQL sequence (SEQ ID NO: 3), present, for example, in gluten peptides such as gliadins.

The protein, composition, or cell of the invention is able to degrade peptides present in gluten. Therefore, another aspect of the invention relates to the *in vitro* use of the protein, composition, or cell of the invention in the degradation of a peptide or fragment thereof present in gluten.

In a preferred embodiment, the peptide is a gliadin, preferably wherein the gliadin is α-gliadin.

In another preferred embodiment, alone or in combination with other preferred embodiments, the peptide or fragment thereof comprises the amino acid sequence SEQ ID NO: 3.

In another preferred embodiment, alone or in combination with other preferred embodiments, the peptide or fragment thereof comprises the sequence SEQ ID NO: 4, which in turn comprises the amino acid sequence SEQ ID NO: 3.

In another preferred embodiment, alone or in combination with other preferred embodiments, the peptide is 33-mer of α-gliadin.

Another aspect of the present invention relates to the use of the protein, composition, or cell of the invention in the treatment of a food, food product, food ingredient or additive that contains gluten, wherein said treatment comprises the degradation of gluten in the food, food product, food ingredient or additive, hereinafter, "the use in the pre-treatment of foods of the invention".

In a preferred embodiment of the use in the pre-treatment of foods of the invention, the gluten in turn comprises gliadins, preferably α-gliadins.

In this aspect of the invention, the term "treatment", referring to a food, food product, food ingredient or additive, is understood to be the processing thereof prior to consumption by a subject, for the purpose of modifying the content or composition of its components, in the present invention gluten, reducing or partially or completely removing same.

Methods or techniques to evaluate whether gluten degradation has occurred include, but are not limited to, the use of ELISA kits for specific antigens derived from gluten intake, such as G12 or R5, spectrophotometric, chromatographic and mass spectrometry techniques, among others
In another aspect, the invention relates to the use of the protein, composition, or cell of the invention in the preparation of a gluten-free food, food product, ingredient or food additive, hereinafter "the use in the preparation of gluten-free foods of the invention".

The term "gluten-free" refers to the fact that the food, food product, food ingredient or additive has gluten levels below 20 ppm. In a preferred embodiment of the present aspect of the invention, the food, food product, food ingredient or additive has gluten levels below 20 ppm. Methods or techniques to determine the levels of gluten in a food or food product, food ingredient or additive, include, but are not limited to, the use of ELISA kits for specific antigens derived from gluten intake, such as G12 or R5, spectrophotometric, chromatographic and mass spectrometry techniques, among others

In theory, any food, food product, food ingredient or additive can be prepared as gluten-free by means of the use of the protein, composition, or cell of the invention.

The terms food product, ingredient or additive have been previously explained herein, where they, as well as their preferred embodiments, are applicable to the present aspect of the invention.

### Technological adjuvant

Since the protein of the invention is able to degrade peptides present in gluten, such as gliadin peptides, can be used as a technological adjuvant in the preparation of gluten-reduced foods. Technological adjuvant is understood to be any substance which is not consumed as a food in itself, but rather is intentionally used in the processing of raw materials, foods or ingredients thereof to fulfil a certain technological purpose during treatment or processing, and may give rise to the unintentional, but technically unavoidable, presence in the end product of residues of the technological adjuvant itself or the derivatives thereof, provided that they do not present any health risk and that they do not have any technological effect on the end product.

Therefore, another aspect of the present invention relates to the use of the protein, the cell of the invention, or the composition of the invention as a technological adjuvant, preferably, in the preparation of foods.

Therefore, the use as a technological adjuvant promotes the degradation of gliadin and immunotoxic peptides in the food or product that are responsible for triggering celiac disease and disorders associated with gluten intake when gluten is consumed.

Another aspect of the present invention relates to the protein, the cell of the invention, or the composition of the invention for use as a technological adjuvant.

### Method of obtaining of the invention

Another aspect of the present invention relates to a method of obtaining the composition of the invention, comprising subjecting a protein comprising the amino acid sequence SEQ ID NO: 2 to a step of mutagenesis, wherein said step of mutagenesis comprises the use of two specific primers the nucleotide sequences of which comprise, preferably consist of, GTGACCTGCAGGTCGTTGATACCTATG (SEQ ID NO: 13) and GCACATAGCTCGCCTTGCCGT (SEQ ID NO: 14).

Techniques for carrying out protein mutagenesis are known to one skilled in the art, including, but not limited to, PCR or the use of Stratagene's QuickChange targeted mutagenesis kit, which are preferred ways of carrying out the step of mutagenesis in the context of the present invention.

The terms used to define the present inventive aspect have been explained in previous inventive aspects, and both they and their preferred embodiments, are applicable to the present inventive aspect.

### DESCRIPTION OF THE FIGURES

**Fig. 1****.** Analysis of the expression and purification of neprosin variants.
**Fig. 2****.** Analysis of neprosin and celiacase activation at pH 3 and 37°C.
**Fig. 3****.** Activity of celiacase, neprosin and mutant variants at 37°C and pH 3 against the fluorogenic peptide MCA-QPQL-Dpa-A-R-NH2.
**Fig. 4****.** Activity of neprosin and celiacase at 37°C and pH 3 against the fluorogenic peptide MCA-QPQL-Dpa-A-R-NH₂, after one week at 4°C, -80°C and lyophilised.

### EXAMPLES

Next, the invention will be illustrated by means of assays carried out by the inventors which demonstrate the effectiveness of the invention.

To design celiacase, first, several mutants were generated and both their activity and their performance during enzyme activation were studied. The synthetic neprosin gene used comes from *Nepenthes ventrata* and is 91% identical to the orthologue of *Nephentes alata* (uniprot ID: A0A1L7NZU4). The coding sequence for the neprosin zymogen was transferred without the original signal peptide (SEQ. ID 12) in the pCMV-sp6-splgk vector that included a signal peptide that would remain at the N-terminus of the celiacase for protein export.

SEQ ID 12:
CGTAGCATTCAGGCGCGTCTGGCGAATAAACCGAAAGGCACCATCAAAACCATCA
AGGGCGACGACGGCGAAGTTGTGGATTGCGTTGACATCTACAAGCAGCCGGCGT
TCGATCACCCGCTGCTGAAAAACCACACCCTGCAGATGCAACCGAGCAGCTATGC
GAGCAAGGTTGGCGAGTACAACAAACTGGAACAACCGTGGCACAAGAACGGCGA
GTGCCCGAAAGGCAGCATCCCGATTCGTCGTCAGGTGATCACCGGTCTGCCGGT
GGTTAAGAAACAATTCCCGAACCTGAAGTTTGCTCCGCCGAGCGCGAACACCAAC
CACCAGTATGCGGTTATTGCGTACTTCTATGGTAACGCGAGCCTGCAAGGCGCGA
ACGCGACCATCAACATTTGGGAGCCGAACCTGAAAAACCCGAACGGTGACTTTAG
CCTGACCCAGATCTGGATTAGCGCGGGTAGCGGCAGCAGCCTGAACACCATTGA
AGCGGGTTGGCAGGTGTATCCGGGTCGTACCGGTGATAGCCAACCGCGTTTCTT
TATTTACTGGACCGCGGACGGTTATACCAGCACCGGCTGCTACGATCTGACCTGC
CCGGGTTTCGTTCAGACCAACAACTACTATGCGATCGGCATGGCGCTGCAACCGA
GCGTGTACGGTGGCCAGCAATATGAGCTGAACGAAAGCATTCAGCGTGACCCGG
CGACCGGTAACTGGTGGCTGTATCTGTGGGGTACCGTGGTTGGCTATTGGCCGG
CGAGCATCTACAACAGCATTACCAACGGTGCGGACACCGTTGAGTGGGGTGGCG
AAATCTACGATAGCAGCGGTACCGGTGGCTTCCACACCACCACCCAAATGGGTAG
CGGCCACTTTCCGACCGAAGGTTACGGCAAGGCGAGCTATGTGCGTGACCTGCA
GTGCGTTGATACCTATGGTAACGTGATCAGCCCGACCGCGAACAGCTTTCAAGGC
ATTGCGCCGGCGCCGAACTGCTACAACTATCAATTTCAGCAGGGCAGCAGCGAA
CTGTACCTGTTTTACGGTGGTCCGGGTTGCCAA

Therefore, the pS6-proNEP construct, which adds a his-tag at the C-terminus (AIAHHHHHH; SEQ ID NO: 5 corresponding to the his-tag sequence) is generated for purification, resulting in the sequence SEQ ID NO: 6

SEQ ID NO: 6
MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGNASLQGANATINIWEPNLKNPNGDFSLTQIWI
SAGSGSSLNTIEAGWQVYPGRTGDSQPRFFIYWTADGYTSTGCYDLTCPGFVQTNN

YYAIGMALQPSVYGGQQYELNESIQRDPATGNWWLYLWGTVVGYWPASIYNSITNG
ADTVEWGGEIYDSSGTGGFHTTTQMGSGHFPTEGYGKASYVRDLQCVDTYGNVISP
TANSFQGIAPAPNCYNYQFQQGSSELYLFYGGPGCQAIAHHHHHH

To generate the different mutants, based on the sequence of neprosin (SEQ ID NO: 2), or from the construct that additionally includes the his-tag sequence at the C-terminus (SEQ ID NO: 5), the following mutations were made: R25K, S26T, I27L, Q28N, R30E, A32S, K36R, T41S, V49M, L65K, H69P, T70S, S76N, E89K, P91S, N95S, K100E, G101V, T111S, L113V, K117R, N161T, N166D, G167K, S182T, S183N, T215S, T217S, T230V, Q241K, V244T, G247A, Q248E, G271S, T272V, G279S, S284T, N287E, T304S, F306Y, T320R, K325R, C334V, V335T, T337E, Y338R, N340Q, V341T, S343T, T345P, A346T, N347E, S348K, N357Q, N360K, Q362E, Q365E, S367K, and S368K.

The XGR3 mutant was synthesised, containing all the mutations listed above. In addition, both in neprosin and XGR3, extra point mutations were made through PCR-based mutagenesis or by the use of Stratagene's QuickChange targeted mutagenesis kit. The final mutants generated are: XGR3, XGR3+N152T, XGR3+N145Q. In addition, the single mutant C334V (celiacase; SEQ ID NO: 1) and the mutants C334V+N145Q, C334V+N152T and C334V+N145Q+N152T, were generated.

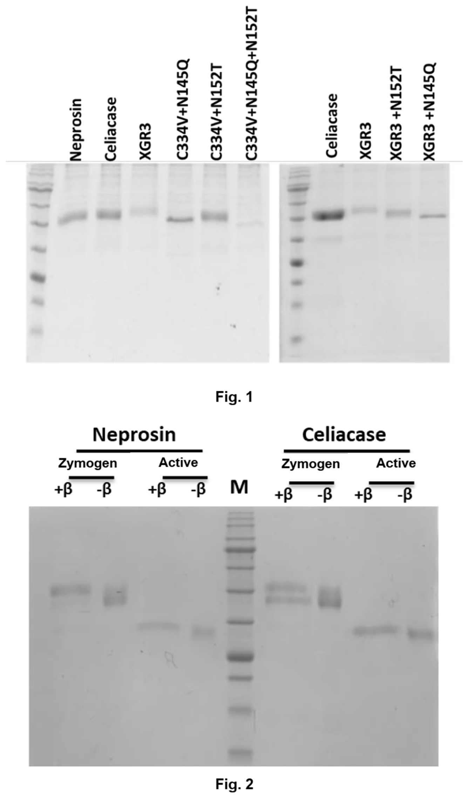

The expression (Fig. 1), activity, and productivity of the different mutants were then evaluated. After analysing the results, mutant C334V (celiacase) showed the best activity, stability, and performance, improving the capabilities of neprosin, the sequence of which is as follows:

MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGNASLQGANATINIWEPNLKNPNGDFSLTQIWI
SAGSGSSLNTIEAGWQVYPGRTGDSQPRFFIYWTADGYTSTGCYDLTCPGFVQTNN
YYAIGMALQPSVYGGQQYELNESIQRDPATGNWWLYLWGTVVGYWPASIYNSITNG
ADTVEWGGEIYDSSGTGGFHTTTQMGSGHFPTEGYGKASYVRDLQVVDTYGNVISP
TANSFQGIAPAPNCYNYQFQQGSSELYLFYGGPGCQ (SEQ ID NO: 1)

For the remaining mutants, the resulting sequence is:
Amino acid sequence of mutant XGR3: 
MQAKFFTFVILSSVFYFNYPLAEAKTLNAELSNKPRGTIKSIKGDDGEMVDCVDIYKQP
AFDHPKLKNPSLQMQPNSYASKVGEYNKLKQSWHKSGECPEVSIPIRRQVISGVPVV
RKQFPNLKFAPPSANTNHQYAVIAYFYGNASLQGANATINIWEPTLKNPDKDFSLTQI
WISAGSGTNLNTIEAGWQVYPGRTGDSQPRFFIYWTADGYSSSGCYDLTCPGFVQV
NNYYAIGMALKPSTYGAEQYELNESIQRDPATGNWWLYLWSVVVGYWPSSIYNTITE
GADTVEWGGEIYDSSGSGGYHTTTQMGSGHFPREGYGRASYVRDLQVTDERGQTIT
PPTEKFQGIAPAPQCYKYEFQEGKKELYLFYGGPGCQ (SEQ ID NO: 7)
Amino acid sequence of mutant XGR3-N145Q-N152T: 
MQAKFFTFVILSSVFYFNYPLAEAKTLNAELSNKPRGTIKSIKGDDGEMVDCVDIYKQP
AFDHPKLKNPSLQMQPNSYASKVGEYNKLKQSWHKSGECPEVSIPIRRQVISGVPVV
RKQFPNLKFAPPSANTNHQYAVIAYFYGQASLQGATATINIWEPTLKNPDKDFSLTQI
WISAGSGTNLNTIEAGWQVYPGRTGDSQPRFFIYWTADGYSSSGCYDLTCPGFVQV
NNYYAIGMALKPSTYGAEQYELNESIQRDPATGNWWLYLWSVVVGYWPSSIYNTITE
GADTVEWGGEIYDSSGSGGYHTTTQMGSGHFPREGYGRASYVRDLQVTDERGQTIT
PPTEKFQGIAPAPQCYKYEFQEGKKELYLFYGGPGCQ (SEQ ID NO: 8)
Amino acid sequence of mutant C334V-N145Q: 
MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGQASLQGANATINIWEPNLKNPNGDFSLTQIWI
SAGSGSSLNTIEAGWQVYPGRTGDSQPRFFIYWTADGYTSTGCYDLTCPGFVQTNN
YYAIGMALQPSVYGGQQYELNESIQRDPATGNWWLYLWGTVVGYWPASIYNSITNG
ADTVEWGGEIYDSSGTGGFHTTTQMGSGHFPTEGYGKASYVRDLQVVDTYGNVISP
TANSFQGIAPAPNCYNYQFQQGSSELYLFYGGPGCQ (SEQ ID NO: 9)
Amino acid sequence of mutant C334V+N152T: 
MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGNASLQGATATINIWEPNLKNPNGDFSLTQIWI
SAGSGSSLNTIEAGWQVYPGRTGDSQPRFFIYWTADGYTSTGCYDLTCPGFVQTNN
YYAIGMALQPSVYGGQQYELNESIQRDPATGNWWLYLWGTVVGYWPASIYNSITNG
 
ADTVEWGGEIYDSSGTGGFHTTTQMGSHFPTEGYGKASYVRDLQVVDTYGNVISP
TANSFQGIAPAPNCYNYQFQQGSSELYLFYGGPGCQ (SEQ ID NO: 10)
Amino acid sequence of mutant C334V+N145Q+N152T:

MQAKFFTFVILSSVFYFNYPLAEARSIQARLANKPKGTIKTIKGDDGEVVDCVDIYKQPA
FDHPLLKNHTLQMQPSSYASKVGEYNKLEQPWHKNGECPKGSIPIRRQVITGLPVVK
KQFPNLKFAPPSANTNHQYAVIAYFYGQASLQGATATINIWEPNLKNPNGDFSLTQIWI
SAGSGSSLNTIEAGWQVYPGRTGDSQPRFFIYWTADGYTSTGCYDLTCPGFVQTNN
YYAIGMALQPSVYGGQQYELNESIQRDPATGNWWLYLWGTVVGYWPASIYNSITNG
ADTVEWGGEIYDSSGTGGFHTTTQMGSGHFPTEGYGKASYVRDLQVVDTYGNVISP
TANSFQGIAPAPNCYNYQFQQGSSELYLFYGGPGCQ (SEQ ID NO: 11)

Both celiacase and the remaining mutants may include the his-tag sequence at the C-terminus (for example, AIAHHHHHH; SEQ ID NO: 5 corresponding to the his-tag sequence) for purification.

Both neprosin and celiacase are produced in their proforma or zymogen state and an acidic pH similar to that of the stomach is necessary (Fig. 2) for the enzyme to change into its active form. To produce the enzyme, the Expi293 human cell line (Thermofisher Scientific) is used, where the recombinant protein is secreted into the medium. These cells are grown at 37°C in the Multitron cell shaker Incubator (Infors HT) at 37°C. The cells are transfected with the DNA plasmid containing neprosin, celiacase or any variant. Once the protein is produced, the medium is clarified by centrifugation and the medium is supplemented with 20 mM imidazole and then subjected to nickel affinity purification (Ni-NTA); finally, the protein is eluted in 300 mM imidazole. Molecular exclusion purification is then performed using a Superdex75 10/300 GL column (GE Healthcare) and the ÄKTA Purifier liquid chromatography system (GE Healthcare). Neprosin, celiacase and the other variants were activated at pH 3.0. To analyse the purity of the samples, both the chromatographic profile of the filtration gel and a 20% SDS-PAGE gel with Coomassie staining were taken into account. Between about 10 mg and 20 mg of protein per litre of culture are obtained from the production yield of both neprosin and celiacase; the remaining variants have a lower yield. After producing both proteins, the amount of enzyme lost during activation at pH=3 was compared, and it was found that less celiacase was lost than neprosin during activation (Fig. 2).

The activity of celiacase and neprosin was evaluated against the fluorogenic peptide MCA-QPQL-Dpa-A-R-NH₂ (Fig. 3). The QPQL sequence (SEQ ID NO: 3) is part of the target that is able to cleave celiacase, which is part of the peptides that generate the celiac disease response in celiac patients, such as 33-mer (LQLQPFPQPQLPYPQPQLPYPQPQLPYPQPQPF; SEQ ID NO: 4), specifically, the enzyme is able to cleave those peptide sequences between P and Q or Y that human digestion enzymes are not able to cleave, as discussed above and which are present in gliadin peptides of different lengths. Celiacase is more effective than neprosin in performing this cleavage reaction (Fig. 3).

To perform activity assays with the fluorogenic peptide MCA-QPQL-Dpa-A-R-NH₂ (GenScript), a concentration of 10 µM of peptide per 100 nM of enzyme, either neprosin, celiacase or the variant, was used. The fluorescence signal was measured during peptide digestion by each enzyme until the curve reached stationary phase. Once the measurement was completed, the slope of the linear area of each activity was calculated. Activity assays with the fluorogenic peptide were performed at 37°C for 30 minutes. All assays were performed in quadruplicate (Fig. 3).

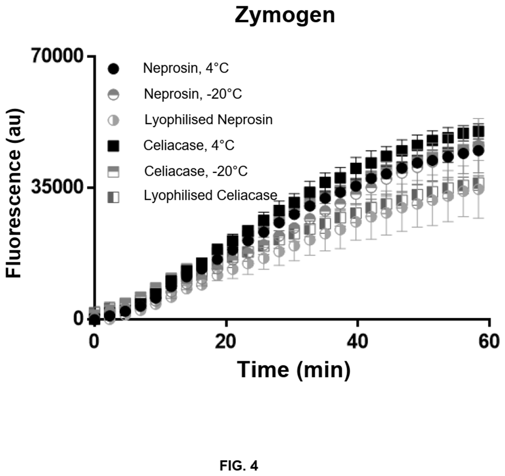

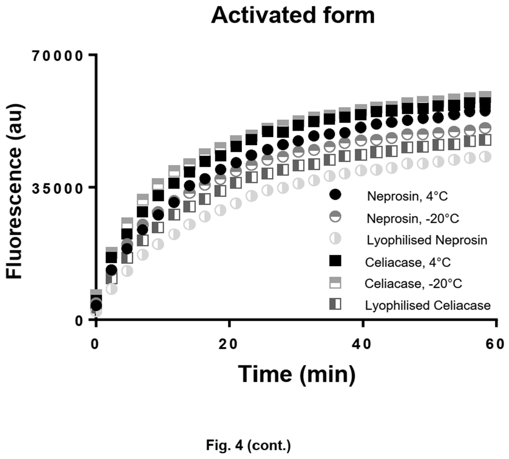

Using the same peptide MCA-QPQL-Dpa-A-R-NH₂ (GenScript) to evaluate stability and storage, protein activity was evaluated at 37°C and pH 3.0, after one week at 4°C, frozen at -80°C or lyophilised (Fig. 4). Both neprosin and celiacase can be stored any of the three processes; in the activated form, celiacase shows a higher recovery of activity after freezing when compared to neprosin. (Fig. 4)

## Claims

1. A protein comprising the amino acid sequence SEQ ID NO: 1.

2. The protein according to claim 1, wherein the protein has a length of 380 to 500 amino acids.

3. The protein according to claim 1, consisting of the amino acid sequence SEQ ID NO: 1.

4. A polynucleotide encoding a protein according to any one of claims 1 to 3.

5. A gene construct comprising a polynucleotide according to claim 4.

6. A vector comprising a polynucleotide according to claim 4, or a gene construct according to claim 5.

7. A cell comprising a protein according to any one of claims 1 to 3, a polynucleotide according to claim 4, a gene construct according to claim 5, or a vector according to claim 6.

8. A composition comprising a protein according to any one of claims 1 to 3, a polynucleotide according to claim 4, a gene construct according to claim 5, a vector according to claim 6, or a cell according to claim 7.

9. A composition according to the preceding claim, further comprising a carrier.

10. The composition according to claim 8 or 9, wherein the composition is a pharmaceutical composition.

11. The composition according to any one of claims 8 to 10, wherein the composition is formulated for oral administration.

12. The composition according to any one of claims 8 to 11, wherein the composition is formulated in solid form or liquid form.

13. The composition according to claim 12, wherein the solid formulation is selected from the list consisting of tablets, pills, chewable tablets, capsules, sachets, powders, gels, granules, coated particles, dispersible strips and films.

14. The composition according to claim 13, wherein the liquid formulation is selected from the list consisting of oral solutions, suspensions, emulsions, drops and syrup.

15. The protein according to any one of claims 1 to 3, a cell according to claim 7, or a composition according to any one of claims 8 to 14, for use thereof as a medicinal product.

16. The protein according to any one of claims 1 to 3, a cell according to claim 7, or a composition according to any one of claims 8 to 14, for use in the prevention, attenuation and/or treatment of a disease due to the presence of peptide antigens in a subject, wherein the peptide antigen is present in gluten.

17. The protein, cell or composition for use according to claim 16, wherein the peptide antigen is a food antigen.

18. The protein, cell or composition for use according to claim 16 or 17, wherein the peptide antigen is present in a gliadin or a fragment thereof, preferably wherein the gliadin is an α-gliadin.

19. The protein, cell or composition for use according to any one of claims 16 to 18, wherein the peptide antigen comprises the amino acid sequence SEQ **ID** NO: 3.

20. The protein, cell or composition for use according to claim 19, wherein the antigen comprises the amino acid sequence SEQ ID NO: 4, which in turn comprises the amino acid sequence SEQ ID NO: 3.

21. The protein, cell or composition for use according to any one of claims 18 to 20, wherein the peptide antigen is 33-mer of α-gliadin or a fragment thereof.

22. The protein, cell or composition for use according to any one of claims 16 to 21, wherein the disease is celiac disease and/or disorders associated with gluten intake.

23. The protein, cell or composition for use according to any one of claims 16 to 22, wherein the disease is a bowel disease.

24. A pharmaceutical product, a veterinary product, a medical food, a food product, a food ingredient or additive, a food supplement, a probiotic food composition, a dietary supplement, a symbiotic composition, or a biotherapeutic composition, comprising an effective amount of the composition according to any one of claims 8 to 14.

25. A pharmaceutical product, a veterinary product, a medical food, a food product, a food supplement, a food ingredient or additive, a probiotic food composition, a dietary supplement, a symbiotic composition, or a biotherapeutic composition according to claim 24, further comprising appropriate amounts of acceptable excipients.

26. Use of a protein according to any one of claims 1 to 3, a cell according to claim 7, or a composition according to any one of claims 8 to 14, as a technological adjuvant.

27. *In vitro* use of a protein according to any one of claims 1 to 3, a cell according to claim 7, or a composition according to any one of claims 8 to 14, in the degradation of a peptide or fragment thereof present in gluten.

28. Use according to claim 27, wherein the peptide is a gliadin, preferably wherein the gliadin is α-gliadin.

29. Use according to claim 27 or 28, wherein the peptide or fragment thereof comprises the amino acid sequence SEQ ID NO: 3.

30. Use according to claim 29, wherein the peptide or fragment thereof comprises the sequence SEQ ID NO: 4, which in turn comprises the amino acid sequence SEQ ID NO: 3.

31. Use according to any one of claims 28 to 30, wherein the peptide is 33-mer of α-gliadin.

32. Use of a protein according to any one of claims 1 to 3, a cell according to claim 7, or a composition according to any one of claims 8 to 14, in the treatment of a food, food product, food ingredient or additive that contains gluten, wherein said treatment comprises the degradation of gluten in the food, food product, food ingredient or additive.

33. Use according to claim 32, wherein the gluten in turn comprises gliadins, preferably α-gliadins.

34. Use of a protein according to any one of claims 1 to 3, a cell according to claim 7, or a composition according to any one of claims 8 to 14, in the preparation of a gluten-free food, food product, food ingredient or additive.

35. A method of obtaining a protein according to any one of claims 1 to 4, comprising subjecting a protein with an amino acid sequence SEQ ID NO: 2 to a step of mutagenesis, wherein said mutagenesis comprises the use of two specific primers the nucleotide sequence of which comprises, preferably consists of, the sequence SEQ ID NO: 13 and SEQ ID NO: 14.
